# EUROPEAN PATENT SPECIFICATION

(11) **EP 2 772 230 B1**
(45) Date of publication and mention of the grant of the patent: **05.07.2017**
(21) Application number: 12844108.6
(22) Date of filing: 12.07.2012
(51) Int. Cl.: A61F 2/44, A61F 2/30

(54) **FUSION PROSTHESIS FOR THE AXIS**
FUSIONSPROTHESE FÜR DEN ZWEITEN HALSWIRBEL
PROTHÈSE DE FUSION POUR L'AXIS

(30) Priority: 24.10.2011 CN 201110326485
(43) Date of publication of application: 03.09.2014
(73) Proprietor: Beijing AK Medical Co., Ltd., Beijing 102200 (CN)
(72) Inventor: LIU, Zhongjun, Beijing 102200 (CN)
(74) Representative: Studio Torta S.p.A.
(86) International application number: PCT/CN2012/078528
(87) International publication number: WO 2013/060168

(56) References cited:
- WO-A1-02/087475
- WO-A2-2005/077039
- WO-A2-2007/038559
- CN-A- 1 053 004
- CN-A- 1 122 686
- CN-A- 101 128 166
- CN-A- 102 319 129
- CN-A- 102 429 747
- CN-A- 102 579 163
- CN-U- 201 996 692
- CN-U- 202 342 236
- US-A- 6 099 531
- US-A1- 2008 215 093
- US-A1- 2010 161 061
- US-A1- 2011 046 741

## Description

### Technical field of the invention

The disclosure relates to the field of orthopaedics implants, in particular to a fusion prosthesis.

### Background of the invention

Spine is the median axis strut of human body, with the upper part connected with the occipital bone at the bottom of skull and the lower part reaching the end of coccyx. Spine is connected with vertebrae, sacra and coccyx with the help of intervertebral discs, intervertebral joints and multiple ligaments so as to be the movement center of trunk and the hub for mechanical transmission and play the roles of supporting weight, absorbing shock, protecting the central nervous system, and providing the movement of anterior and/or posterior flexion and extension, lateral bending, twisting and so forth for the upper body. Due to the change of modern lifestyles and accelerated pace of life, the load on the spine and its surrounding tissues is increased, resulting in a higher damage possibility and increased spine diseases occurrence rate. The common diseases related to spine, such as vertebral damage and loss caused by trauma, tumor, congenital malformation, tuberculosis, rheumatoid arthritis and other lesions, occur occasionally, and the current solutions for the loss mainly include bone grafting filling, artificial vertebral body replacement, etc., supplemented by the internal fixation of screw plates and screw rods in the front or back.

However, the problem of prior art is that the fusion implant need to maintain a pressure with its close upper and lower physiological vertebrae to prevent dislocation. Therefore it is necessary to implant another rigid screw plate or screw rod system to keep the pressure. But, usually, such fixing system is higher than the surface of an original physiological vertebral body, so as to cause adverse effect on the surrounding nerves, blood supplies and soft tissues. In addition, with quite different elasticity modulus the rigid fixing system and the physiological bone cannot be fused together, so fatigue fracture happens easily for a long-term use.

Specifically, it should be mentioned that, for some vertebral joints at a specific stage, such as the atlanto-axial joint which is a special and important spinal structure for connecting skull and spine, they cannot be replaced by the ordinary artificial ones of cervical spine due to their special position and anatomical structure once they are lost because of lesion, and there is no alternative of prosthesis implantation currently. In clinic, doctors can only temporarily put autogenous bone or allogeneic bone into modified titanium cage or titanium case for the bone graft fusion based on specific cases. And the patients have to suffer a long-time entire head-neck-should fixation until the fusion is complete, which is very inconvenient.

US 2008/0215093 A1 discloses a degradable cage coated with mineral layers for spinal interbody fusion, in one form of which, the cage includes a plurality of spaced apart walls comprising a biodegradable polymeric material (e.g., polycaprolactone); an osteoconductive mineral coating (e.g., a calcium compound) on at least a portion of the walls; and a bioactive agent (e.g., a bone morphogenetic protein) associated with the polymeric material and/or the coating.

WO 2005/077039 A2 provides orthopedic implants which are at least partially absorbable. The implants of the invention may include a biocompatible material in the form of a ring, which may be used in combination with a second, more porous, absorbable material. This second material may be a continuous body or discontinuous.

US 6099531 A discloses a changing relationship between bones, wherein a wedge member is moved into a joint between the bones. As the wedge member enters the joint, pivotal movement occurs between the bones to change the orientation of the bones relative to each other. The wedge member may have a circular cross sectional configuration and be moved into the joint by rotating the wedge member about an axis which extends between a thin leading edge portion and a thick trailing edge portion of the wedge The porous material of which the wedge member may be made has the properties specified in the preamble of present claim 1. US 2010/161061 A1 discloses an implant for interfacing with a bone structure which includes a web structure including a space truss. The space truss includes two or more planar truss units having a plurality of struts joined at nodes and the web structure is configured to interface with human bone tissue.

WO 02/087475 A1 discloses an improved ceramic bone graft for human implantation, particularly such as a spinal fusion cage (10) for implantation into the intervertebral space between two adjacent vertebrae. The improved spinal fusion cage (10) includes a substrate block of high strength ceramic having a selected size and shape to fit the anatomical space, and a controlled porosity analogous to natural bone. The substrate block is coated with a bio-active surface coating material such as hydroxyapatite or a calcium phosphate to promote bone ingrowth and enhanced bone fusion.

### Summary of the invention

The objective of this disclosure is to provide a fusion prosthesis according to claim 1 which can replace the bone structure of a human body for implantation.

The fusion prosthesis according to this disclosure is arranged between the first and second bone structures to replace the bone structure between them and comprises a prosthesis body and prosthesis fusion surfaces on the prosthesis body, wherein the prosthesis fusion surfaces are inosculated with at least one of the first and second bone structures and are provided with microporous structures.

Furthermore, the microporous structures are multidirectional microporous structures comprising a plurality of micropores communicated with one another.

Furthermore, the aperture of pores of the microporous structures is within 100 to 1,800 microns.

Furthermore, the fusion prosthesis is an axis fusion prosthesis to replace the axis between an atlas and a third cervical vertebra, and the prosthesis fusion surfaces comprise upper joint fusion surfaces arranged at the upper end of the prosthesis body and inosculated with the atlas.

Furthermore, the axis fusion prosthesis may be configured such that after it is implanted, its front surface is in the front physiological surface of an original axis.

Furthermore, the prosthesis body may be provided with a plurality of third connection pores which are inclined and upwards extended, and the axis fusion prosthesis is connected with the atlas and the third cervical vertebra respectively through fasteners.

Furthermore, an odentoid boss may be arranged on the upper joint fusion surfaces of the prosthesis body corresponding to the odontoid of the original axis.

Furthermore, vertical plates may be arranged at the upper end of the prosthesis body opposite respectively to the lateral masses outside anterior arch of the atlas, and clamp the anterior arch of the atlas together with the odontoid bosses.

Furthermore, there may be two vertical plates opposite to the left and right lateral masses outside the anterior arch of the atlas respectively, and each is provided with a first connection pore.

Furthermore, the prosthesis fusion surfaces may comprise lower joint fusion surfaces located at the lower end of the prosthesis body and inosculated with the third cervical vertebra.

Furthermore, the lower joint fusion surfaces may be provided with one or more second connection pores.

Furthermore, the prosthesis body may be provided with reinforced parts on its surface and/or in its interior.

Furthermore, the fusion prosthesis may be arranged between the atlas and the third cervical vertebra to replace the axis, and is configured to be identical to the original physiological axis joint in the total height or extended downwards to replace the axis and the third cervical vertebra or more segments of cervical vertebra after being implanted.

Furthermore, the prosthesis body is a microporous structure. Furthermore, the prosthesis body is provided with a plurality of bone grafting pores which are penetrated or communicated with one another through the microporous structures.

Furthermore, the aperture of the bone grafting pores may be within 2 to 30 mm.

Through the fusion prosthesis of the disclosure, the prosthesis body is a metal implant of equal size manufactured according to the tomography data, such as CT, MRI, and UCT, of a patient. The fusion prosthesis is provided with the prosthesis fusion surfaces which are constructed according to the replaced bone structures and are well inosculated with at least one of the first and second bone structures. The prosthesis fusion surfaces are microporous structures favorable for the ingrowth of bone cells. This microporous structures are multidirectional microporous structures communicated with one another, and the pores are favorable for the crawling and ingrowth of bone cells. After an operation, the prosthesis fusion surfaces of the fusion prosthesis will be fused with the bone articular surface of at least one of the first and second bone structures to achieve the long-term stability.

### Brief description of the drawings

The drawings constituting one part of the application are to provide further understanding of the disclosure, and the exemplary embodiments of the disclosure and the explanations thereof are intended to explain the disclosure, instead of improperly limiting the disclosure. In the drawings:
Fig. 1 is a diagram showing the solid structure of a fusion prosthesis of the disclosure;
Fig. 2 is a diagram showing the microporous structures of the fusion prosthesis of the disclosure;
Fig. 3 is a diagram showing the structures of the fusion prosthesis of the disclosure connected with and disassembled from an axis;
Fig. 4 is a diagram showing the structure of the fusion prosthesis of the disclosure connected with an axis through screws;
Fig. 5 is a diagram showing the structure of the fusion prosthesis of the disclosure disassembled from screws;
Fig. 6 is a diagram showing the structure of bone grafting pores of the fusion prosthesis of the disclosure;
Fig. 7 is a diagram showing the extension structure of the fusion prosthesis in one embodiment of the disclosure; and
Fig. 8 is a diagram showing the application state of the extension structure of the fusion prosthesis in one embodiment of the disclosure.

### Detailed description of the invention

The disclosure will be described below in detail with reference to the drawings and in conjunction with the embodiments.

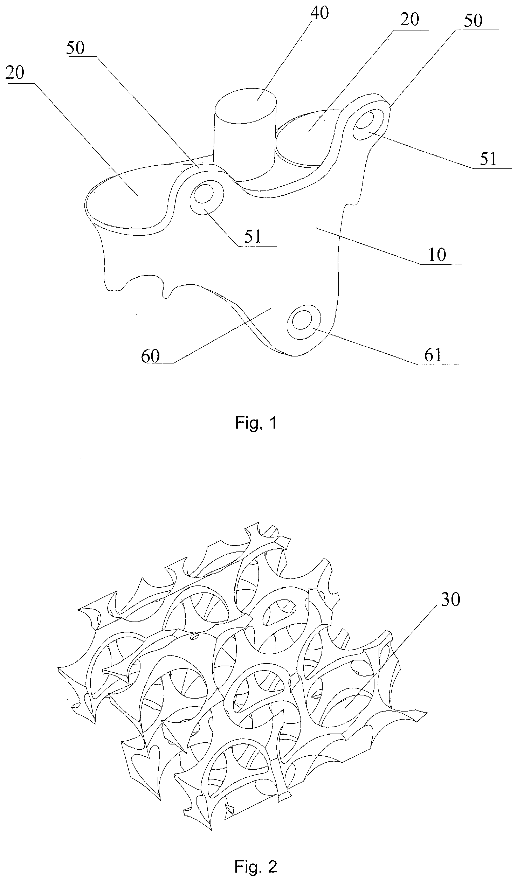

As shown in Fig. 1, a fusion prosthesis 1 according to this disclosure is arranged between the first and second bone structures 2, 3 to replace the bone structure between them and comprises a prosthesis body 10 and prosthesis fusion surfaces on the prosthesis body 10, wherein the prosthesis fusion surfaces are inosculated with at least one of the first and second bone structures 2, 3, and the surfaces of the prosthesis fusion surfaces are microporous structures which are a plurality of multidirectional microprorous structures communicated with one another.

Through the fusion prosthesis 1 of the disclosure, the prosthesis body 10 is a medical metal implant of equal size manufactured according to the tomography data, such as CT, MRI, and UCT, of a patient, and the fusion prosthesis 1 is made of medical metals so as to have good biocompatibility. The fusion prosthesis 1 is provided with prosthesis fusion surfaces which are constructed according to a replaced bone structure and are well inosculated with at least one of the first and second bone structures 2, 3. The prosthesis fusion surfaces are microporous structures 30 favorable for the ingrowth of bone cells, and the porous diameter of the microporous structures 30 is whthin 100 to 1,800 microns. As shown in Fig. 2, the microporous structures 30 are multidirectional microporous structures communicated with one another, and their pores are favorable for the crawling and ingrowth of bone cells. After an operation, the prosthesis fusion surfaces of the fusion prosthesis 1 will be fused with the bone articular surface of at least one of the first and second bone structures 2, 3 to achieve the long-term stability. Preferably, the prosthesis fusion surfaces, which are inosculated with the first and second bone structures 2, 3, are arranged at the two ends of the fusion prosthesis 1 respectively.

Preferably, the prosthesis body 10 is a microporous structure made of medical metals and the pores on its surface and inside it are penetrated with one another, with an aperture of 100 to 1,800 microns.

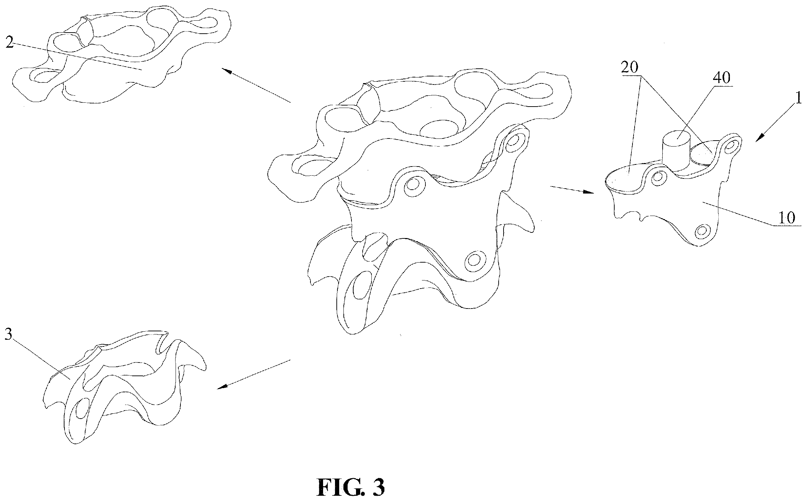

As shown in Fig. 3, the fusion prosthesis 1 according to the disclosure is an axis fusion prosthesis arranged between the first and second bone structures 2, 3 to replace an axis. In this embodiment, the first bone structure 2 is an atlas, and the second bone structure 3 is a third cervical vertebra. The prosthesis fusion surfaces comprise upper joint fusion surfaces 20 at the upper end of the prosthesis body 10 and inoculated with the atlas. Preferably, the prosthesis fusion surfaces comprise lower joint fusion surfaces 60 at the lower end of the prosthesis body 10 and inosculated with the third cervical vertebra.

Out of necessity for operative approach, the axis fusion prosthesis may not have the transverse, spinous or vertebral arch structure, and is basically identical to the size of the original physiological axis of a patient except for the necessary change of functional design. The axis fusion prosthesis is provided with the upper joint fusion surfaces 20 and the lower joint fusion surfaces 60 at the upper and lower ends respectively. The upper and the lower joint fusion surfaces 20 and 60 are processed by a numerical control machine after extracting the curve data of the lower articular surface of the atlas and the upper articular surface of third cervical vertebra respectively, and can also be provided with the high curve fitting characteristic by fast forming technology such as laser sintering or high-energy electronic beam melting. The upper joint fusion surfaces 20 of the fusion prosthesis are manufactured according to the data of the lower articular surface of the atlas and are well inosculated with the lower articular surface of the atlas, and the lower joint fusion surfaces 60 of the fusion prosthesis are manufactured according to the data of the upper articular surface of third cervical vertebra and are well inosculated with the upper articular surface of third cervical vertebra. After an operation, the upper and the lower joint fusion surfaces 20 and 60 of the axis fusion prosthesis will be inosculated with the bone articular surfaces of the atlas and the third cervical vertebra to achieve the long-term stability because their surfaces are both the microporous structures.

As shown in the Fig. 1, according to one embodiment of the axis fusion prosthesis, the upper joint fusion surfaces 20 of the prosthesis body 10 are provided with an odontoid boss 40 opposite to the odontoids of the original axis. Vertical plates 50 are arranged at the upper end of the prosthesis body 10 opposite to the lateral masses outside anterior arch of the atlas and clamp the anterior arch of the atlas together with the odontoid boss 40. Preferably, there are two vertical plates 50 opposite to the left and right lateral masses outside the anterior arch of the atlas, and each is provided with a first connection pore 51.

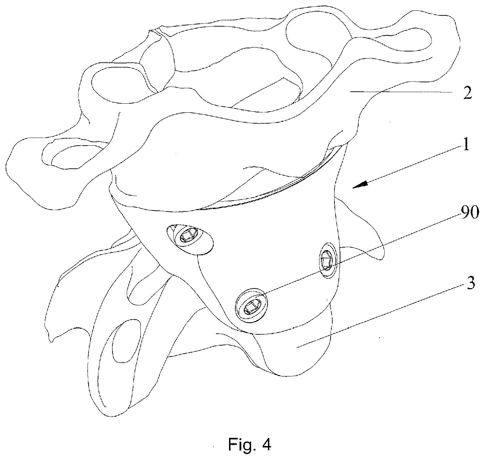

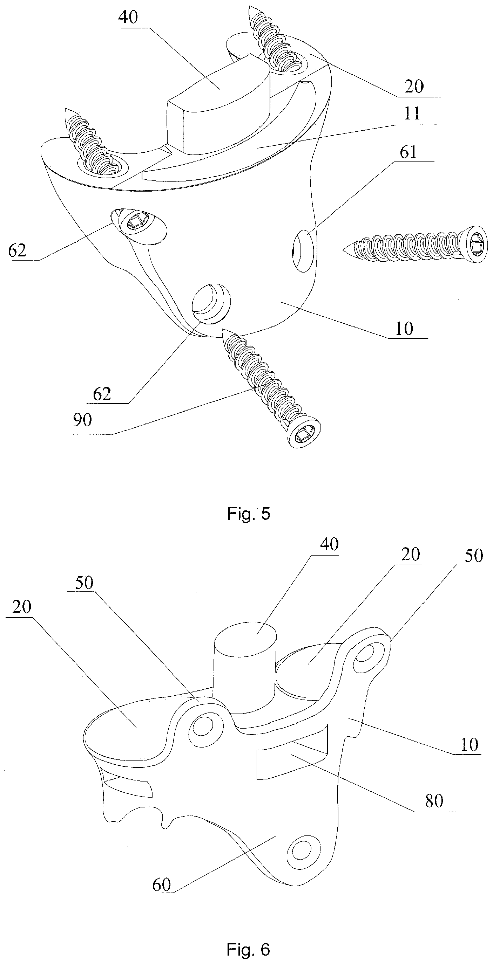

The outer surfaces of the odontoid boss 40 and the inner surfaces of the vertical plates 50 contacted with the lateral masses of the atlas are the microporous structures 30 favorable for the ingrowth of bone cells. The microporous structures 30 are multidirectional microporous structures communicated with one another, with a porous diameter of 100 to 1,800 microns, and the pores are favorable for the crawling and ingrowth of bone cells. After an operation, the odontoid boss 40 and the microporous structures on inner surfaces of the vertical plates 50 are fused with the odontoid fovea inside the anterior arch of the atlas and the lateral masses of the atlas respectively to combine the fusion prosthesis with the atlas and achieve the long-term stability. As shown in Figs. 4 and 5, in the operation, the connectors 90 are screwed in the lateral masses of the atlas through two first connection pores 51 to achieve the initial stability, and then the stable reconstruction of cervical vertebra can be implemented after the bone fusion is completed. In this embodiment, the connectors 90 are fixing screws.

According to another embodiment of the axis fusion prosthesis, as shown in Fig. 5, the axis fusion prosthesis is configured such that after it is implanted, its front surface is in the front physiological surface of the original axis.

In this embodiment, a recess 11 is formed at the upper end of the axis fusion prosthesis opposite to the inferior margin of anterior arch of the atlas, and the upper end of the axis fusion prosthesis is well inoculated with the inferior margin of anterior arch of the atlas. The prosthesis body 10 is provided with a plurality of third connection pores 62 which are inclined and extended upwards, and the axis fusion prosthesis is connected with the atlas and the third cervical vertebra respectively through the fasteners 90. Specifically, the third connection pores 62 are screw pores, and the fasteners 90 are fixing screws. Two screw pores are formed below the left and right lateral masses outside the anterior arch of the atlas, and, in the operation, the fixing screws are screwed in the lateral masses of the atlas though the screw pores to achieve the initial stability. The surface of the recess 11 arranged at the upper end of prosthesis body 10 of the axis fusion prosthesis opposite to the inferior margin of the anterior arch of the atlas and contacted with the inferior margin of the atlas is microporous structures. The microporous structures are multidirectional microporous structures communicated with one another, with a porous diameter of 100 to 1,800 microns, and the pores are favorable for the crawling and ingrowth of bone cells. After an operation, the microporous structures on the inner surface of the recess 11 arranged at the upper end of prosthesis body 10 of the axis fusion prosthesis opposite to the inferior margin of the anterior arch of the atlas will be fused with the bone in the inferior margin of anterior arch of the atlas to achieve the long-term stability.

In this embodiment, after the axis fusion prosthesis is implanted, its front surface is in the front physiological surface of the original axis, i.e., not projected from the front physiological surface of the original axis, also called zero incisura medically. Such design is favourable in that the front surfaces of the atlas, the axis fusion prosthesis, and the third cervical vertebra form an almost continuous cervical vertebra front surface with no obvious projections after the operation, and no adverse influence is caused to the dissection positions of various soft tissues in front of the axis, thereby avoiding the feeling of foreign body and discomfort of a patient during breath, swallowing and communications caused by the fixation of the routine cervical vertebra front steel plate in which the implanted artificial axis part is remarkably projected from the front surface of the original physiological axis.

As shown in Fig.1, the lower joint fusion surfaces 60 are provided with one or more second connection pores 61.

According to one embodiment of the disclosure, the prosthesis fusion surfaces at the lower end of the prosthesis body 10 opposite to the upper articular surface of the third cervical vertebra are microporoous structures favorable for the ingrowth of bone cells, and the pores are favorable for the crawling and ingrowth of bone cells. After an operation, the prosthesis fusion surfaces at the lower end of the axis fusion prosthesis opposite to the upper articular surface of the third cervical vertebra will be fused with the upper articular surface of the third cervical vertebra to achieve the long-term stability. The prosthesis fusion surfaces at the lower end of the axis fusion prosthesis opposite to the upper articular surface of the third cervical vertebra are provided with 1 to 3 screw pores. As shown in Figs. 4 and 5, in the operation, the fixing screws 90 are screwed in the upper articular surface of the third cervical vertebra through second connection pores 61 respectively to achieve the initial stability and then the stable reconstruction of cervical vertebra can be implemented after the bone fusion is completed.

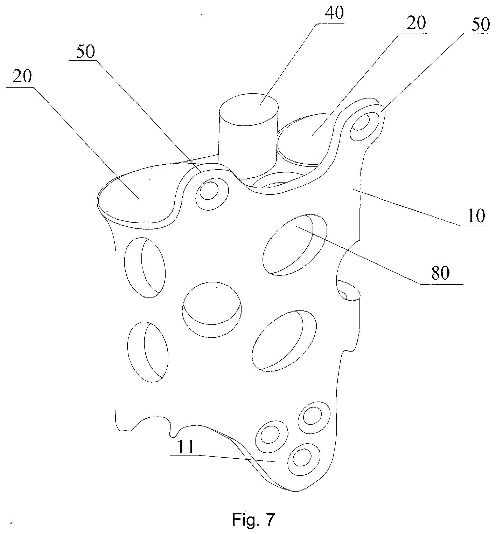

As shown in Figs. 6 and 7, the prosthesis body 10 is provided with a plurality of bone grafting pores 80 which are penetrated with one another or communicated with one another through the microporous structures.

A number of bone grafting pores 80 are formed on the prosthesis body of the axis fusion prosthesis, which can accommodate autogenous or allogeneic bones and bone granules. Before or in an operation, autogenous or allogeneic bones and bone granules are implanted in these bone grafting pores 80 to induce the crawling or ingrowth of bone cells to promote the bone fusion. The aperture of these bone grafting pores is within 2 to 30 mm to facilitate the implantation of autogenous or allogeneic bones and bone granules with different diameters.

The surface and/or inside of the prosthesis body of the fusion prosthesis is/are completely or partly applied with hydroxyapatite coatings which are formed by plasma spraying coating or electrochemical deposition and sintering.

In addition, the axis fusion prosthesis is arranged between the atlas and the third cervical vertebra to replace the axis, and is identical to the original physiological axis joint in the total height after being implanted.

The surface and/or inside of the prosthesis body 10 is/are provided with reinforced parts which are reinforced ribs or reinforced plates to reinforce the structural strength of the prosthesis body 10.

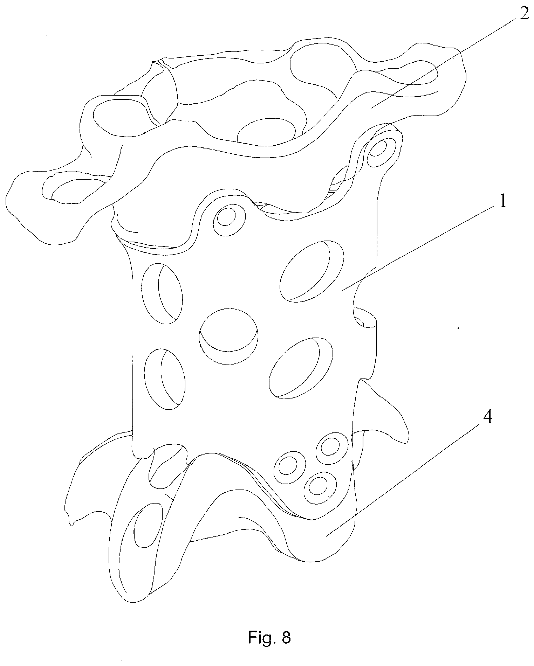

As shown in Figs. 7 and 8, when the axis and third cervical vertebra or more cervical vertebra segments of a patient need to be replaced by the prosthesis, the fusion prosthesis is extended downwards to cover and replace the third cervical vertebra 4 and more cervical vertebra segments. At this point, the fusion prosthesis will be inoculated and fixed with the fourth or other cervical vertebra, and the fixing fusion way is the same as the butting fixation way of the third cervical vertebra.

The method for processing the axis fusion prosthesis is: such tomography data as CT, MRI, and UCT of cervical vertebra of a patient is acquired, and a 3D data model of diseased region of cervical vertebra is established in a computer, then the axis model is extracted and modified and designed to generate a 3D data model of the axis fusion prosthesis, then the physiological axis is stimulated and replaced with the 3D data model of the axis fusion prosthesis to check and verify the design scheme, and the fusion prosthesis is processed and formed.

According to the existing processing technology, there are two ideal ways for processing:
A, the fusion prosthesis is processed and formed by a numerical control machine according to the processing procedure converted by the data of the axis fusion prosthesis generated in the computer, and the required bone grafting pores, screw pores and micropores are drilled and milled by electric spark machining, chemical corrosion, mechanical drilling and cutting, and the like, and fixing screws are made by ordinary mechanical processing, such as turning or milling;
B, the fusion prosthesis is formed by fusion by fast forming technology, such as laser sintering or high-energy electronic beam melting, specifically as follows:
   a), the 3D data model of the axis fusion prosthesis designed and constructed in the computer is layered by professional software to obtain the outline data of a series of single-layer slices;
   b), the data of the series of slices is input to the laser or high-energy electronic beam fast forming equipment;
   c), medical metal powder, the thickness of which corresponds to the layering height of the 3D data model, is laid in the processing chamber of the laser or high-energy electronic beam fast forming equipment;
   d), the medical metal powder is scanned and selectively melt by the laser or high-energy electronic beam under the control of a computer;
   e), the steps of laying and scanning and melting the powder are repeated to sinter the materials selected to be melt in each layer into a whole;
   f), after the melting is completed on all layers, the powder not molten is removed to obtain the axis fusion prosthesis in the required shape;
   g), the various structures above will be made at a time in the process of laser sintering or high-energy electronic beam melting because such structures as entities, bone grafting pores, screw pores and micropores have been designed in the data files during the construction of 3D data model of the axis fusion prosthesis.

It can be seen from what described above that the embodiments of the disclosure have the following technical effects:
For the fusion prosthesis of the disclosure, the prosthesis fusion surfaces are microporous structures favorable for the ingrowth of bone cells. The microporous structures are multidirectional microporous structures communicated with one another, and the pores are favorable for the crawling and ingrowth of bone cells. After an operation, the prosthesis fusion surfaces of the fusion prosthesis will be fused with the bone articular surface of at least one of the first and second bone structures to achieve the long-term stability

What said above are only the preferred embodiments of the disclosure and not intended to limit the disclosure. For those skilled in the art, various modifications and changes can be made in the disclosure. Any modifications, equivalent replacements, improvements and the like within the claims shall fall within the scope of protection of the disclosure.

## Claims

1. A fusion prosthesis, which is configured to be arranged between a first bone structure (2) and a second bone structure (3) to replace bone structure between the first and second bone structures (2, 3); the fusion prosthesis comprises a prosthesis body (10) and prosthesis fusion surfaces on the prosthesis body (10); and the prosthesis fusion surfaces are configured to be constructed according to a bone structure to be replaced and inosculated with at least one of the first and second bone structures (2, 3) and are provided with microporous structures (30); the prosthesis body (10) is a microporous structure, the prosthesis body (10) is provided with a plurality of bone grafting pores (80) which communicate with one another through the microporous structures and are configured to accommodate autogenous or allogeneic bones and bone granules;
the microporous structures (30) are multidirectional microporous structures comprising a plurality of micropores communicated with one another; the porous aperture of the microporous structures (30) is within 100 to 1, 800 microns;
the fusion prosthesis is an axis fusion prosthesis for replacing the axis between an atlas and a third cervical vertebra, and the prosthesis fusion surfaces comprise upper joint fusion surfaces (20) arranged at the upper end of the prosthesis body (10) and inosculated with the atlas;
**characterized in that**, an odontoid boss (40) is arranged on the upper joint fusion surfaces (20) of the prosthesis body (10) to correspond to the odontoid of an original axis.

2. The fusion prosthesis according to claim 1, **characterized in that**, the axis fusion prosthesis is configured such that after it is implanted, its front surface is in the front physiological surface of the original axis.

3. The fusion prosthesis according to claim 2, **characterized in that**, the prosthesis body (10) is provided with a plurality of third connection pores (62) which are inclined and extended upwards, and the axis fusion prosthesis is connected with the atlas and the third cervical vertebra respectively through fasteners (90).

4. The fusion prosthesis according to claim 1, **characterized in that**, vertical plates (50) are arranged at the upper end of the prosthesis body (10) opposite to the lateral masses outside the anterior arch of the atlas and clamp the anterior arch of the atlas together with the odontoid bosses (40).

5. The fusion prosthesis according to claim 4, **characterized in that**, there are two vertical plates (50) opposite respectively to the left and right lateral masses outside the anterior arch of the atlas, and each vertical plate (50) is provided with a first connection pore (51).

6. The fusion prosthesis according to claim 1, **characterized in that**, the prosthesis fusion surfaces further comprise lower joint fusion surfaces (60) located at the lower end of the prosthesis body (10) and inosculated with the third cervical vertebra.

7. The fusion prosthesis according to claim 6, **characterized in that**, the lower joint fusion surfaces (60) are provided with one or more second connection pores (61).

8. The fusion prosthesis according to claim 1, **characterized in that**, the prosthesis body (10) is provided with reinforced parts on its surface and/or in its interior.

9. The fusion prosthesis according to claim 1, **characterized in that**, the fusion prosthesis is arranged between the atlas and the third cervical vertebra to replace the axis, and is configured to be identical to the original physiological axis joint in the total height or extended downwards to replace the axis and the third cervical vertebra or more segments of cervical vertebra after being implanted.

10. The fusion prosthesis according to claim 1, **characterized in that**, the aperture of the bone grafting pores (80) is within 2 to 30 mm.

## Patentansprüche

1. Eine Fusionsprothese, die dazu ausgelegt ist, zwischen einer ersten Knochenstruktur (2) und einer zweiten Knochenstruktur (3) angeordnet zu werden, um Knochenstruktur zwischen den ersten und zweiten Knochenstrukturen (2, 3) zu ersetzen; die Fusionsprothese umfasst einen Prothesenkörper (10) und Prothesen-Fusionsoberflächen an dem Prothesenkörper (10); und die Prothesen-Fusionsoberflächen sind dazu ausgelegt, gemäß einer zu ersetzenden Knochenstruktur aufgebaut zu werden und mit wenigstens einer der ersten und zweiten Knochenstrukturen (2, 3) inoskuliert zu werden und mit mikroporösen Strukturen (30) versehen sind; der Prothesenkörper (10) ist eine mikroporöse Struktur, der Prothesenkörper (10) ist mit mehreren Knochenaufbau-Poren (80) versehen, die miteinander durch die mikroporösen Strukturen in Verbindung stehen und dazu ausgelegt sind, autogene oder allogene Knochen und Knochenkörnchen aufzunehmen;
die mikroporösen Strukturen (30) sind multidirektionale mikroporöse Strukturen, umfassend mehrere miteinander in Verbindung stehende Mikroporen; die Porenöffnung der mikroporösen Strukturen (30) ist zwischen 100 und 1800 mm;
die Fusionsprothese ist eine Axis-Fusionsprothese zum Ersetzen des zweiten Halswirbels (Axis) zwischen einem Atlas und einem dritten Halswirbel und die Prothesen-Fusionsoberflächen umfassen obere Gelenk-Fusionsoberflächen (20), die an dem oberen Ende des Prothesenkörpers (10) angeordnet und mit dem Atlas inoskuliert sind;
**dadurch gekennzeichnet, dass** ein Dornvorsprung (40) an den oberen Gelenk-Fusionsoberflächen (20) des Prothesenkörpers (10) angeordnet ist, um dem Dorn eines ursprünglichen Axis zu entsprechen.

2. Fusionsprothese gemäß Anspruch 1, **dadurch gekennzeichnet, dass** die Axis-Fusionsprothese so ausgelegt ist, dass nach ihrer Implantation ihre vordere Oberfläche in der vorderen physiologischen Oberfläche des ursprünglichen Axis ist.

3. Fusionsprothese gemäß Anspruch 2, **dadurch gekennzeichnet, dass** der Prothesenkörper (10) mit mehreren dritten Verbindungsporen (62) versehen ist, die geneigt sind und sich nach oben erstrecken und die Axis-Fusionsprothese mit dem Atlas bzw. dem dritten Halswirbel durch Befestigungsmittel (90) verbunden ist.

4. Fusionsprothese gemäß Anspruch 1, **dadurch gekennzeichnet, dass** vertikale Platten (50) an dem oberen Ende des Prothesenkörpers (10) gegenüber den lateralen Massen außerhalb des vorderen Bogens des Atlas angeordnet sind und den vorderen Bogen des Atlas mit den Dornvorsprüngen (40) zusammenklemmen.

5. Fusionsprothese gemäß Anspruch 4, **dadurch gekennzeichnet, dass** zwei vertikale Platten (50) sich gegenüber der linken bzw. rechten lateralen Masse außerhalb des vorderen Bogens des Atlas befinden und jede vertikale Platte (50) mit einer ersten Verbindungspore (51) versehen ist.

6. Fusionsprothese gemäß Anspruch 1, **dadurch gekennzeichnet, dass** die Prothesen-Fusionsoberflächen ferner untere Gelenk-Fusionsoberflächen (60) umfassen, die an dem unteren Ende des Prothesenkörpers (10) angeordnet und mit dem dritten Halswirbel inoskuliert sind.

7. Fusionsprothese gemäß Anspruch 6, **dadurch gekennzeichnet, dass** die unteren Gelenk-Fusionsoberflächen (60) mit einer oder mehreren zweiten Verbindungsporen (61) versehen sind.

8. Fusionsprothese gemäß Anspruch 1, **dadurch gekennzeichnet, dass** der Prothesenkörper (10) mit verstärkten Teilen an seiner Oberfläche und/oder in seinem Inneren versehen ist.

9. Fusionsprothese gemäß Anspruch 1, **dadurch gekennzeichnet, dass** die Fusionsprothese zwischen dem Atlas und dem dritten Halswirbel angeordnet ist, um den Axis zu ersetzen und dazu ausgelegt ist, mit dem ursprünglichen physiologischen Axis-Gelenk in der gesamten Höhe identisch zu sein oder sich abwärts zu erstrecken, um den Axis und den dritten Halswirbel oder mehr Segmente der Halswirbelsäule nach Implantation zu ersetzen.

10. Fusionsprothese gemäß Anspruch 1, **dadurch gekennzeichnet, dass** die Öffnung der Knochenaufbau-Poren (80) zwischen 2 und 30 mm ist.

## Revendications

1. Prothèse de fusion, qui est configurée pour être disposée entre une première structure osseuse (2) et une seconde structure osseuse (3) afin de remplacer une structure osseuse entre les première et seconde structures osseuses (2, 3) ; la prothèse de fusion comprenant un corps de prothèse (10) et des surfaces de fusion de prothèse sur le corps de prothèse (10) ; et les surfaces de fusion de prothèse étant configurées pour être construites en fonction d'une structure osseuse à remplacer et jointes à au moins une des première et seconde structures osseuses (2, 3) et étant pourvues de structures microporeuses (30) ; le corps de prothèse (10) étant une structure microporeuse, le corps de prothèse (10) étant pourvu d'une pluralité d'orifices de greffe osseuse (80) qui communiquent les uns avec les autres par le biais des structures microporeuses et sont configurés pour recevoir des os et des granules osseux autogènes ou allogéniques ;
les structures microporeuses (30) étant des structures microporeuses multidirectionnelles comprenant une pluralité de micropores reliés les uns aux autres ; l'ouverture de pore des structures microporeuses (30) étant comprise entre 100 et 1800 micromètres ;
la prothèse de fusion étant une prothèse de fusion d'axis servant à remplacer l'axis entre un atlas et une troisième vertèbre cervicale, et les surfaces de fusion de prothèse comprenant des surfaces de fusion d'articulation supérieures (20) disposées au niveau d'une extrémité supérieure du corps de prothèse (10) et jointes à l'atlas ; **caractérisée en ce qu'**un bossage odontoïde (40) est disposé sur les surfaces de fusion d'articulation supérieures (20) du corps de prothèse (10) de façon à correspondre à l'odontoïde d'un axis d'origine.

2. Prothèse de fusion selon la revendication 1, **caractérisée en ce que** la prothèse de fusion d'axis est configurée de telle sorte qu'après son implantation, sa surface avant se trouve dans la surface physiologique avant de l'axis d'origine.

3. Prothèse de fusion selon la revendication 2, **caractérisée en ce que** le corps de prothèse (10) est pourvu d'une pluralité de troisièmes orifices de raccordement (62) qui sont inclinés et s'étendent vers le haut, et la prothèse de fusion d'axis est respectivement raccordée à l'atlas et à la troisième vertèbre cervicale par le biais d'éléments de fixation (90).

4. Prothèse de fusion selon la revendication 1, **caractérisée en ce que** des plaques verticales (50) sont disposées au niveau de l'extrémité supérieure du corps de prothèse (10) face aux masses latérales à l'extérieur de l'arc antérieur de l'atlas et fixent l'arc antérieur de l'atlas conjointement avec les bossages odontoïdes (40).

5. Prothèse de fusion selon la revendication 4, **caractérisée en ce que** deux plaques verticales (50) sont présentes respectivement face aux masses latérales gauche et droite à l'extérieur de l'arc antérieur de l'atlas, et chaque plaque verticale (50) est pourvue d'un premier orifice de raccordement (51).

6. Prothèse de fusion selon la revendication 1, **caractérisée en ce que** les surfaces de fusion de prothèse comprennent en outre des surfaces de fusion d'articulation inférieures (60) situées au niveau de l'extrémité inférieure du corps de prothèse (10) et jointes à la troisième vertèbre cervicale.

7. Prothèse de fusion selon la revendication 6, **caractérisée en ce que** les surfaces de fusion d'articulation inférieures (60) sont pourvues d'un ou de plusieurs deuxièmes orifices de raccordement (61).

8. Prothèse de fusion selon la revendication 1, **caractérisée en ce que** le corps de prothèse (10) est pourvu de parties renforcées sur sa surface et/ou dans sa partie intérieure.

9. Prothèse de fusion selon la revendication 1, **caractérisée en ce que** la prothèse de fusion est disposée entre l'atlas et la troisième vertèbre cervicale afin de remplacer l'axis, et est configurée pour être identique à l'articulation d'axis physiologique d'origine du point de vue de la hauteur totale ou prolongé vers le bas afin de remplacer l'axis et la troisième vertèbre cervicale ou davantage de segments de vertèbres cervicales après son implantation.

10. Prothèse de fusion selon la revendication 1, **caractérisée en ce que** l'ouverture des orifices de greffe osseuse (80) est comprise entre 2 et 30 mm.
